# EUROPEAN PATENT APPLICATION

(11) **EP 2 354 132 A2**
(43) Date of publication of application: **10.08.2011**
(21) Application number: 10305124.9
(22) Date of filing: 05.02.2010
(51) Int. Cl.: C07D 307/80

(54) **Crystallized form of dronedarone base**

(71) Applicant: SANOFI, 75013 Paris (FR)
(72) Inventor: The designation of the inventor has not yet been filed
(74) Representative: Romanowski, Caroline

(57) **Abstract**

The invention relates to a novel crystallized form of dronedarone base, to its process of preparation and to the pharmaceutical compositions containing said novel crystallized form.

## Description

The instant invention relates to a crystallized form of dronedarone base and to its process of preparation.

2-n-butyl-3-[4-(3-di-n-butylaminopropoxy)benzoyl]-5-methylsulfonamido-benzofuran, or dronedarone, and its pharmaceutically acceptable salts are described in the European patent EP 0 471 609 B1.

Dronedarone hydrochloride is an antiarrhythmic drug indicated to reduce the risk of cardiovascular hospitalization in patients with a history of, or current atrial fibrillation. This drug, commercialized under the tradename Multaq^{®}, has been approved by the Health authorities since mid-2009 in the form of a tablet containing the hydrochloride salt of dronedarone. As usual in the course of pharmaceutical research and development, investigations to discover new forms of dronedarone are on-going, with the aim to identify the most suitable forms of this drug substance for various pharmaceuticals uses and formulations.

In the course of crystallization studies, the Inventors have identified three different crystalline phases for dronedarone in the form of the base, hereafter identified as forms A, B and C, form B being the thermodynamically most stable one. Form B of dronedarone base is more suitable for the use in drug products than the previously known amorphous compound and than the other crystalline phases.

The invention therefore relates to the crystallized form B of dronedarone base, characterized in that its powder X-ray diffractogram shows the following characteristic peaks expressed as interplanar distances at approximately: 18.6 ; 4.75 ; and 4.23 Å (Angström).

In another embodiment, the powder X-ray diffractogram of the crystallized form B of dronedarone base shows the following characteristic peaks expressed as interplanar distances at approximately : 18.6 ; 7.97 ; 6.45 ; 5.73 ; 4.75 ; 4.71 ; 4.65 ; 4.23 ; 4.18; and 3.90 Å.

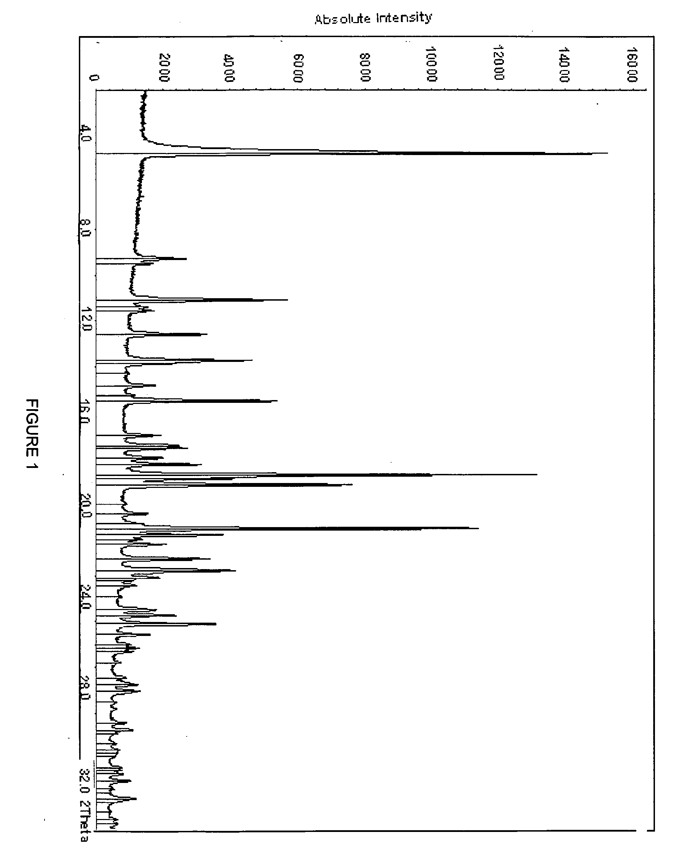

The powder X-ray diffractogram of the crystallized form B of dronedarone base is illustrated in Figure 1. The invention therefore also relates to said form B, characterized by the powder X-ray diffractogram according to Figure 1.

The invention further relates to a crystallized form of dronedarone base (form B) having a melting point of 72°C ± 2°C (corresponding to a DSC peak temperature, as it will be described in more details below).

The invention further relates to a crystallized form of dronedarone base (form B) having a monoclinic unit cell with cell parameters a =19.342 (3) A, b = 8.7930 (24) A, c =19.533 (3) A, β= 105.963 (13) degrees and V = 3193.9 (8) Å³ wherein a, b and c represent the interplanar distances in the cell, β represents the angles within the cell and V represents the volume of the unit cell.

The invention also relates to a process for the preparation of the crystallized form B of dronedarone base, comprising the following steps starting from dronedarone base :
1) preparing the crystallized form A of dronedarone base, and
2) converting said form A into form B by one the following steps :
   a) maturation of a suspension of form A,
   b) addition of an antisolvent to a solution of form A,
   c) evaporation at elevated temperature of a solution or suspension of form A, or
   d) cooling of a solution of form A.

In this process, it shall be understood that the terms « a solution of form A » designates a solution of dronedarone base obtained from form A of dronedarone base. Indeed, once it is solubilized, there is not any more solid in the medium, so neither any crystallized form.

More specifically, the conversion of form A into form B of dronedarone base is carried out by one of the following steps :
a) maturation of a suspension of form A in a solvent or mixture of solvents chosen from water, water/methanol, ethanol, 1-propanol, 2-propanol, 2-butanol, i-butanol, t-butanol, 1-pentanol, acetone, methyl ethyl ketone, i-butyl methyl ketone, ethyl formate, methyl acetate, propyl acetate, i-propyl acetate, butyl acetate, i-butyl acetate, tetrahydrofuran, diisopropyl ether, methyl t-butyl ether, toluene, acetonitrile, methylene chloride, 1,4-dioxane and chloroform (crystallization by maturation of a suspension);
b) dissolving form A in a solvent, then adding an antisolvent (crystallization by adding an antisolvent) ;
c) dissolving or suspending form A in a solvent at elevated temperature, then stirring the solution and allowing the solvent to evaporate from the stirred solution at this elevated temperature (crystallization by evaporation at elevated temperature) ; or
d) dissolving form A in a solvent at elevated temperature, then slowly cooling the solvent to a low temperature (crystallization by cooling of a solution).

In step a) above, the term « suspension » designates form A being mixed with a solvent, and wherein at least a portion of form A remains in a solid state (also designated as a « slurry »).

Step a) is advantageously carried out at about 20°C.

In step a), the maturation time should be sufficient for the crystals to appear in the reaction medium. The maturation time may last several weeks, such as 5 or 6 weeks, or a few days if seeding is used to trigger the crystallization reaction.

In an embodiment of the invention, the solvent used in step b) is chosen from diisopropyl ether and methyl tert-butyl ether.

In step b) above, the term « antisolvent » designates a solvent of low polarity, different from the solvent where form A is dissolved. The addition of the antisolvent enables to precipitate the dronedarone base in the crystallized form B. Said form B is recovered from the reaction medium after filtration, such as vacuum filtration.

In an embodiment of the invention, the antisolvent used in step b) is chosen from alkane solvents, such as pentane, hexane, heptane, cyclohexane or methylcyclohexane. The antisolvent used in step b) is advantageously n-heptane.

Step b) is advantageously carried out at or around room temperature.

In step c) above, the solvent is chosen so as to produce a solution or a suspension of dronedarone base. Said solvent is advantageously chosen from water or a mixture of water and methanol.

In step c) above, « elevated temperature » designates a temperature of about 40°C or more. Advantageously, the temperature in step c) is comprised between about 40°C and about 65°C.

In step d) above, « elevated temperature » designates a temperature of about 40°C or more. Advantageously, the elevated temperature in step d) is comprised between about 40°C and about 80°C. It can be for example of about 50°C.

In step d) above, « low temperature » designates a temperature of about 10°C.

In step d) above, « slowly » cooling designates the cooling of the solution from the elevated temperature to the low temperature in about 24 hours.

In another embodiment of step d) above, the solvent is advantageously chosen among the solvents and mixtures of solvents defined in relation to step a), optionally in the presence of an antisolvent such as n-heptane. Mention may be made in particular of tetrahydrofuran or diisopropyl ether, or of a mixture diisopropyl ether/n-heptane.

Alternatively, the process according to the invention can also be carried out by seeding, i.e. by using seeds of form B of dronedarone base obtained previously. Seeding is a crystallization technique well-known in the Art and will be illustrated in details below.

In another embodiment, the invention therefore relates to a process for the preparation of the crystallized form B of dronedarone base, comprising a step of adding seeds of form B to a reaction medium comprising a solution or suspension of dronedarone base.

The process of crystallization according to the invention enables to obtain the active ingredient dronedarone with very high purity : typically, dronedarone base crystallized according to the process of the invention is obtained with a sum of impurities below 0.3 % (by mass).

The sum of impurities is determined using liquid chromatography. The detector is a UV spectrophotometer working at 246 nm. The stationary phase is a column filled with cyano phase (length = 250 mm ; internal diameter = 4.6 mm ; particle size = 5 µm). The mobile phase consists of a gradient between acetonitrile and a phosphate buffer (pH = 4.0). The flow rate is 0.8 ml/min. The gradient is described in table 1.

**Table 1**

| Time | Acetonitrile | Phosphate buffer pH 4 |
|---|---|---|
| (min.) | (volumes) | (volumes) |
| 0 | 30 | 70 |
| 15 | 40 | 60 |
| 25 | 40 | 60 |
| 40 | 50 | 50 |
| 45 | 60 | 40 |
| 58 | 60 | 40 |
| 60 | 30 | 70 |
| 70 | 30 | 70 |

In view of the increased purity of the active ingredient dronedarone obtained by the crystallization process according to the invention, the instant invention therefore also relates to a process for the purification of dronedarone base, which comprises a step for crystallizing phase B of dronedarone base. In this regard, crystallization may be carried out according to any of the above processes or by using a crystallization procedure with seeding, which is particularly adapted for the industrial level.

The invention also relates to a pharmaceutical composition comprising the crystalline form B of dronedarone base as active ingredient, and at least one pharmaceutically acceptable excipient.

The invention is illustrated by the following examples, which describe in details processes for the preparation of the crystallized form B of dronedarone base, as well as physico-chemical analyses thereof and comparisons with the other crystallized forms A and C of dronedarone base. The following figures are referred to :
- Figure 1 represents the powder X-ray diffractogram of the crystallized form B of dronedarone base,
- Figure 2 represents the Raman spectrum of the crystallized form B of dronedarone base,
- Figure 3 represents the DSC (Differential Scanning Calorimetry) thermogram of the crystallized form B of dronedarone base,
- Figure 4 represents the TGA (Thermogravimetric Analysis) of the crystallized form B of dronedarone base, and
- Figure 5 represents the DVS (Dynamic Vapor Sorption) isotherms of the crystallized form B of dronedarone base.

### I - Processes of preparation

Reproducible crystallization processes have been developed in small and larger scales, with or without seeding. Some examples are detailed below. In each experiment below, the obtention of dronedarone base under the crystalline form B is confirmed by X-Ray Powder Diffraction analyses.

### Example 1 : Preparation of the crystallized form A of dronedarone base

The starting material may be either dronedarone base or the hydrochloride salt thereof, both described in EP 0 471 609 B1. In the latter case, the base may itself be obtained from dronedarone hydrochloride (return to the base form from the salt form).

Dronedarone base in the form of an oil, obtained from dronedarone hydrochloride, is put in solution in a mixture ethanol/water (75%/25% V/V) with a concentration of at least 100 g/L. The solution is heated and maintained under reflux for about 30 minutes, until no more particle is visible in the reaction medium. The temperature inside the reactor is lowered to 25°C as fast as possible and seeding is carried out at this temperature. The seed corresponding to the dronedarone base is introduced under dry state at a concentration of about 1 to 3 % (by weight). The seed was obtained at low temperature (4°C) by contacting dronedarone base, in the form of an oil, with a small quantity of water for 8 hours, without stirring.

This seeding step is followed by an isotherm at 25°C for 30 minutes, then by a cooling from 25°C to at least 12°C, even 5°C, at a speed of 10°C/hour. An isotherm is carried out at the temperature of the end of crystallization for at least 14 hours.

A microscopy analysis of the crystals in suspension before filtration shows that the crystals have the shape of fine sticks.

The solid product is isolated by filtration under vacuum at room temperature. The product is washed with a mixture ethanol/water (2/1 to 3/1, V/V), then dried under vacuum at 30-40°C for at least 24 hours. A solid of white color is obtained, with a yield up to 92%.

This crystalline form of the dronedarone base is designated as form A and has been characterized as follows.

NMR analysis has demonstrated that the solid obtained is dronedarone base (Bruker Advance 300 MHz equiped by a bbiz probe ; analysis carried out at 300K in deuterated chloroform).

X-ray powder diffraction has confirmed that the compound is obtained in a crystallized state (Bruker AXS D8 Advance).

DSC analysis (heating rate : 10°C/min. ; heating from 25°C to at least 150°C) has demonstrated that the form A of dronedarone base transforms to another crystalline form, designated as form C (solid-solid transition at an onset of about 54°C) ; on further heating, form C melts at about 65°C (onset temperature).

X-ray diffractograms registered at various temperatures from room temperature to 100°C, at intervals of 10°C, have confirmed the solid-solid transition from form A to form C. A first crystalline solid phase, designated as form A, exists from room temperature to about 40°C. The diffractogram registered at 50°C reveals the appearance of new peaks and the disappearance of others, consequently the appearance of a new crystalline phase which remains until about 60°C, temperature at which the intensity of some peaks diminishes to evolve gradually towards an amorphous phase (fusion). Two crystalline forms are thus observed : form A from room temperature to about 50°C, and form C appearing above 50°C and melting between 60 and 70°C.

These X-ray analyses demonstrate that the crystalline form of dronedarone base which was described in EP 0 471 609, with a melting point of 65.3°C, corresponds to the form now designated under form A, which transforms in phase C and then melts at the melting point of form C.

### Example 2 : Preparation of the crystallized form B of dronedarone base

The crystallized form A of dronedarone base obtained in example 1 is used as starting material.

### Example 2.1: Preparation of the crystallized form B of dronedarone base by maturation of a suspension of form A

0.202 g of form A of dronedarone base are suspended in 1 ml of water and the suspension is stirred for 5 weeks at 20°C under exclusion of light. The solid material is then isolated by vacuum filtration, dried in vacuum and consists of form B.

The same protocol is performed starting from the following materials, leading to form B as summarized in table 2.

**Table 2**

| Form A of dronedarone base | Suspension medium | Maturation time |
|---|---|---|
| 0.201 g | 1.3 ml water/methanol (1/1 V/V) | 5 weeks |
| 0.496 g | 0.3 ml ethanol | 6 weeks |
| 0.489 g | 0.3 ml 1-propanol | 6 weeks |
| 0.310 g | 0.3 ml 2-propanol | 5 weeks |
| 0.434 g | 0.3 ml 2-butanol | 5 weeks |
| 0.427 g | 0.3 ml iso-butanol | 5 weeks |
| 0.419 g | 0.3 ml tert-butanol | 5 weeks |
| 0.312 g | 0.3 ml 1-pentanol | 5 weeks |
| 0.498 g | 0.3 ml of iso-butyl methyl ketone | 6 weeks |
| 0.512 g | 0.3 ml of propyl acetate | 6 weeks |
| 0.507 g | 0.3 ml iso-propyl acetate | 6 weeks |
| 0.499 g | 0.3 ml butyl acetate | 6 weeks |
| 0.458 g | 0.3 ml iso-butyl acetate | 6 weeks |
| 0.202 g | 0.4 ml diisopropyl ether | 5 weeks |
| 0.395 g | 0.3 ml methyl tert-butyl ether | 6 weeks |

0.576 g of form A of dronedarone base are suspended in 0.3 ml of acetone and the suspension is stirred for 3 weeks at 20°C under exclusion of light. The clear, yellow sample was allowed to evaporate at room temperature for 6 days. The sample showed a wax-like but crystalline appearance and was dried under vacuum at 40°C for 3 days, leading to form B.

The same protocol is performed starting from the following materials, leading to form B as summarized in table 3.

**Table 3**

| Form A of | Suspension medium |
|---|---|
| dronedarone base | (0.3 ml) |
| 0.516 g | methyl ethyl ketone |
| 0.538 g | ethyl formate |
| 0.521 g | methyl acetate |
| 0.502 g | tetrahydrofuran |
| 0.520 g | toluene |
| 0.489 g | acetonitrile |
| 0.484 g | methylene chloride |

### Example 2.2 : Preparation of the crystallized form B of dronedarone base by addition of an antisolvent

- 0.2 g of form A of dronedarone base are dissolved in 0.7 ml of diisopropyl ether at room temperature and, after about 30 minutes, 5 ml of n-heptane are added to the stirred solution to precipitate the product. The precipitate is isolated by vacuum filtration and consists of form B.
- Using the same protocol with 0.5 ml of methyl t-butyl ether as solvent, form B is obtained as well.

### Example 2.3 : Preparation of the crystallized form B of dronedarone base by evaporation at elevated temperature

- 0.201 g of form A of dronedarone base are suspended in 24 ml of water at about 40°C. The solvent is then allowed to evaporate from the stirred solution at the same temperature to obtain form B of dronedarone base.
- 0.206 g of form A of dronedarone base are dissolved in 10 ml of a mixture water/methanol (1/1) at about 65°C. The solvent is then allowed to evaporate from the stirred solution at the same temperature to obtain form B of dronedarone base.

### Example 2.4: Preparation of the crystallized form B of dronedarone base by cooling of a solution

- 0.204 g of form A of dronedarone base are dissolved in 0.3 ml of diisopropyl ether at about 50°C and then cooled to 10°C in 24 h. Since no precipitation occurred, the solvent was allowed to evaporate at room temperature for 4 days to obtain form B of dronedarone base.
- 0.205 g of form A of dronedarone base are dissolved in 20 ml of tetrahydrofuran at about 50°C and then cooled to 10°C in 24 h. Since no precipitation occurred, the solvent was allowed to evaporate at room temperature for 4 days. The sticky sample was then dried at 40°C overnight to obtain form B of dronedarone base.

### Example 3 : Preparation of the crystallized form B of dronedarone base with seeding

### Example 3.1 :

0.5 g of form A of dronedarone base are suspended in 0.8 ml of diisopropyl ether, seeded with a small amount of form B and the suspension is stirred for 4 days at 20°C under exclusion of light. The solid material is then isolated by vacuum filtration and consists of form B.

### Example 3.2 :

49.30 g of form A of dronedarone base are dissolved in a mixture of diisopropyl ether/n-heptane (48%/52%, V/V) at 80°C with a stirring rate of 170 rpm. Dronedarone initial concentration is 176 g/L. Once dissolution is observed, the medium is cooled as quickly as possible until 30°C. At this temperature, seeding (0.2% w/w) is performed with seeds resulting from a small scale crystallisation according to conditions described in example 2.2 (dissolution in diisopropyl ether). After a stabilisation of these seeds at 30°C during 15-20 minutes, a new cooling phase is programmed until 10°C at a rate of about 5°C/h, followed by an isothermal period of about 1 hour. The solid obtained in the medium is isolated by vacuum filtration and washed with 100 ml of n-heptane at ambient temperature. Crystals are dried under oven vacuum at 40°C during 48 h. The yield for the obtention of form B of dronedarone base is 79%.

### Example 3.3 :

362.11 g of form A of dronedarone base are dissolved in a mixture of diisopropyl ether/n-heptane (50%/50%, V/V) at 80°C with a stirring rate of 242 rpm. Dronedarone initial concentration is 170 g/L. Once dissolution is observed, the medium is cooled as quickly as possible until 30°C. At this temperature, seeding (0.8% w/w) is performed with seeds resulting from a small scale crystallisation according to conditions described in example 2.2 (dissolution in diisopropyl ether). After a stabilisation of these seeds at 30°C during 15-20 minutes, a new cooling phase is programmed until 10°C at a rate of about 5°C/h, followed by an isothermal period of about 12 hours. The solid obtained in the medium is isolated by vacuum filtration and washed with 3 x 120 ml of n-heptane at ambient temperature. Crystals are dried under oven vacuum at 40°C during 72 h. The yield for the obtention of form B of dronedarone base is 86%.

### Example 3.4:

11,1 kg of dronedarone hydrochloride are dissolved in methyl t-butyl ether (MTBE) at 50°C. A aqueous solution of potassium carbonate is added to generate the base. The reaction mixture is stirred for 2 hours. The 2 phases are separated. The organic phase is washed 2 times with water and then concentrated up to 0.7 volume of MTBE. 4 volumes of n-heptane are added and the mixture is cooled down to 30°C. A seed is added at 30°C, followed by a isothermal period of 16 hours. The mixture is cooled down to 10°C at a rate of -5°C/h and remains at 10°C for 1 hour. The base is then filtered, washed and dried. The yield is 92,3 %.

### II - Physico-chemical analyses

### 2.1 : Powder X-Ray diffraction

X-ray powder diffraction is performed with Stoe Stadi-P transmission diffractometers using Cu-Kα1 radiation. Linear position sensitive detectors are used and the sample is investigated in a flat preparation. Generator settings for the X-ray tube are 45kV and 35mA. The data is collected in the 2-Theta range from 2 to 34°. The position sensitive detector is moved over this 2-Theta range in steps of 0.2° and intensities are collected for 60 seconds at each position. The measured data is evaluated and plotted with the Software WinXPOW V2.23.

Figure 1, wherein the absolute intensity is plotted against the 2-Theta angle, represents the powder X-ray diffractogram thus obtained for the crystallized form B of dronedarone base, using a dry sample. The diffraction peaks confirm the crystallized state of the product. The peaks whose relative intensity is greater than 25% are summarized in table 4, wherein :
- D represents the interplanar distance, expressed in Angström,
- 2-theta represents the Bragg angle, expressed in degree,
- I/I₀ represents the relative intensity, expressed as a percentage of the most intense peak (Strong: 100% > I > 75%; Medium: 75% ≥ I ≥ 25%).

**Table 4**

| Interplanar distance | 2-Theta angle | I/I₀ |
|---|---|---|
| D (Å) | (°) | |
| 18.6 (± 0.4) | 4.7 (± 0.2) | Strong |
| 7.97 (± 0.07) | 11.1 (± 0.2) | Medium |
| 6.45 (± 0.05) | 13.7 (± 0.2) | Medium |
| 5.73 (± 0.04) | 15.5 (± 0.2) | Medium |
| 4.75 (± 0.03) | 18.7 (± 0.2) | Strong |
| 4.71 (± 0.02) | 18.8 (± 0.2) | Medium |
| 4.65 (± 0.02) | 19.1 (± 0.2) | Medium |
| 4.23 (± 0.02) | 21.0 (± 0.2) | Strong |
| 4.18 (± 0.02) | 21.2 (± 0.2) | Medium |
| 3.90 (± 0.02) | 22.8 (± 0.2) | Medium |

Table 5 provides a comparison of the X-Ray diffractograms of forms A and B of dronedarone base (peaks whose relative intensity is greater than 25%).

**Table 5**

| Form A | | Form B | |
|---|---|---|---|
| 2-Theta angle | I/I₀ | 2-Theta angle | I/I₀ |
| (°) | | (°) | |
| 2.7 (± 0.2) | Medium | 4.7 (± 0.2) | Strong |
| 5.4 (± 0.2) | Medium | 11.1 (± 0.2) | Medium |
| 8.1 (± 0.2) | Medium | 13.7 (± 0.2) | Medium |
| 10.9 (± 0.2) | Medium | 15.5 (± 0.2) | Medium |
| 17.9 (± 0.2) | Medium | 18.7 (± 0.2) | Strong |
| 19.0 (± 0.2) | Medium | 18.8 (± 0.2) | Medium |
| 19.8 (± 0.2) | Strong | 19.1 (± 0.2) | Medium |
| 20.2 (± 0.2) | Medium | 21.0 (± 0.2) | Strong |
| 2 0.4 (± 0.2) | Medium | 21.2 (± 0.2) | Medium |
| 21.3 (± 0.2) | Medium | 22.8 (± 0.2) | Medium |

### 2.2 : Raman spectroscopy

Raman spectra are recorded with an FT-Raman spectrometer (RFS-100/S, Bruker) equipped with a 1.5W NIR-Laser (Nd:YAG; λ=1064 nm) and a nitrogen-cooled D418-T NIR-Detector. The spectra are evaluated and plotted by the software OPUS V. 6.5.

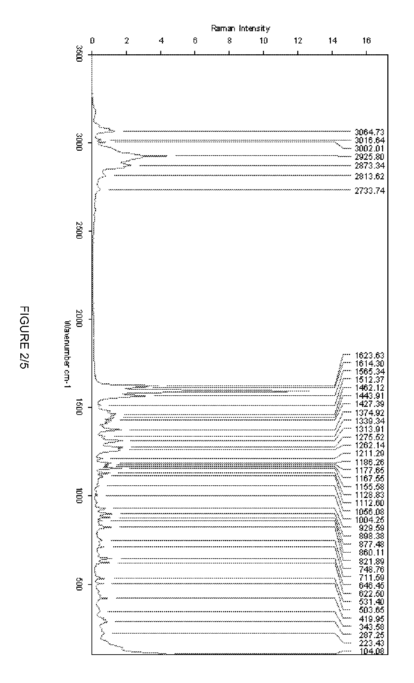

The Raman spectrum of the crystallized form B of dronedarone base is represented on Figure 2, wherein the Raman intensity is plotted against the wavenumber (cm⁻¹).

Table 6 summarizes the Raman key spectral modes thus obtained for forms A and B of dronedarone base.

**Table 6**

| Form A | | Form B | |
|---|---|---|---|
| Wavenumbers | Relative intensity | Wavenumbers | Relative intensity |
| (cm⁻¹) | | (cm⁻¹) | |
| 3083 (± 3) | Weak | 3065 (± 3) | Weak |
| 2936 (± 3) | Medium | 2926 (± 3) | Medium |
| 2914 (± 3) | Medium | | |
| 2873 (± 3) | Medium | 2873 (± 3) | Weak |
| 1625 (± 3) | Medium | 1624 (± 3) | Medium |
| 1601 (± 3) | Medium | 1592 (± 3) | Strong |
| 1555 (± 3) | Strong | 1565 (± 3) | Medium |
| 1435 (± 3) | Medium | 1375 (± 3) | Weak |
| 1372 (± 3) | Medium | 1314 (± 3) | Weak |
| 1263 (± 3) | Medium | 1156 (± 3) | Weak |

| | | | |
|---|---|---|---|
| Strong: 100% > I > 75% ; Medium: 75% ≥ I ≥ 25% ; Weak: I < 25% | | | |

### 2.3 : Differential Scanning Calorimetry (DSC) and Thermogravimetric Analysis (TGA)

All DSC measurements are performed with a Mettler DSC822e (module DSC822e/700/109/414935/0025). If not indicated differently, 40 µL Al-crucibles with sealed lid and hole are used. All measurements are carried out in a nitrogen gas flow of 50 mL/min and chosen heating rate was 10°C/min., with a heating from 25°C to at least 150°C.

The thermogravimetric analyses are performed with a Mettler TGA851e (module TGA/SDTA851e/SF1100/042). 80 µl Al-crucibles with lid and pinhole are used and the measurements are performed in a nitrogen gas flow of 50 mL/min.

The measured data are evaluated via the software STARe V8.10.

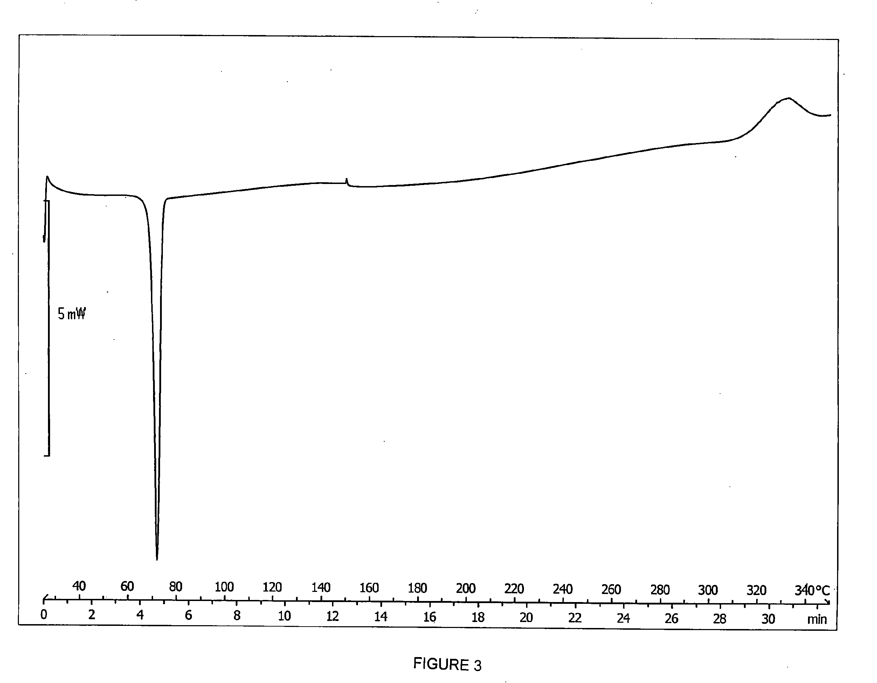

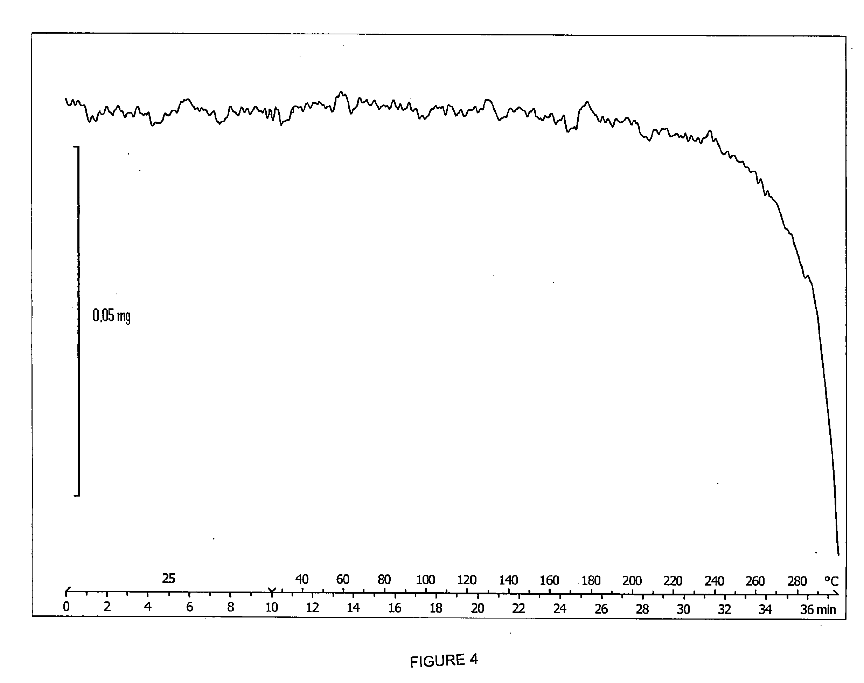

The DSC thermogram of the crystallized form B of dronedarone base is represented on Figure 3, wherein temperature (°C) and time (min.) are plotted. On heating with a rate of 10°C/min, the product melts with an onset temperature of about 69°C. The thermogram denotes a melting point of 72°C ± 2°C. The TG analysis represented on Figure 4, wherein temperature (°C) and time (min.) are plotted, shows that there is no weight loss at the melting point measured under DSC.

In comparison, the crystallized form C of dronedarone base presents a melting point at about 69°C. On heating with a rate of 10°C/min, form A transforms to form C at about 54°C ; on further heating, form C melts at about 65°C (onset temperature of the second endothermic event). After formation of form C, if the heating is stopped below form C melting point and the medium is then cooled back to room temperature, form C typically transforms back to form A between 50 and 45°C.

Table 7 summarizes the DSC data of forms A, B and C of dronedarone base, measured at a heating rate of 10°C/min.

**Table 7**

| **Form A** | | **Form B** | |
|---|---|---|---|
| *1st endothermic event* | | *Endothermic event* | |
| *(leading to **Form C**)* | | | |
| Onset temperature | 54 (± 2) °C | Onset temperature | 69 (± 2) °C |
| Peak temperature | 56 (± 2) °C | Peak temperature | 72 (± 2) °C |
| *2^{nd} endothermic event* | | / | |
| Onset temperature | 65 (± 2) °C | | |
| Peak temperature | 69 (± 2) °C | | |

### 2.4 : Dynamic Vapor Sorption (DVS)

Moisture sorption/desorption isotherms are recorded on a DVS-1 from Surface Measurement Systems. Two cycles are run at 25°C, in which the relative humidity is stepped from 0 to 95% and back to 0%. The complete measurement time for both cycles is about 35 h. The data is evaluated using the software DVSWin V. 2.15.

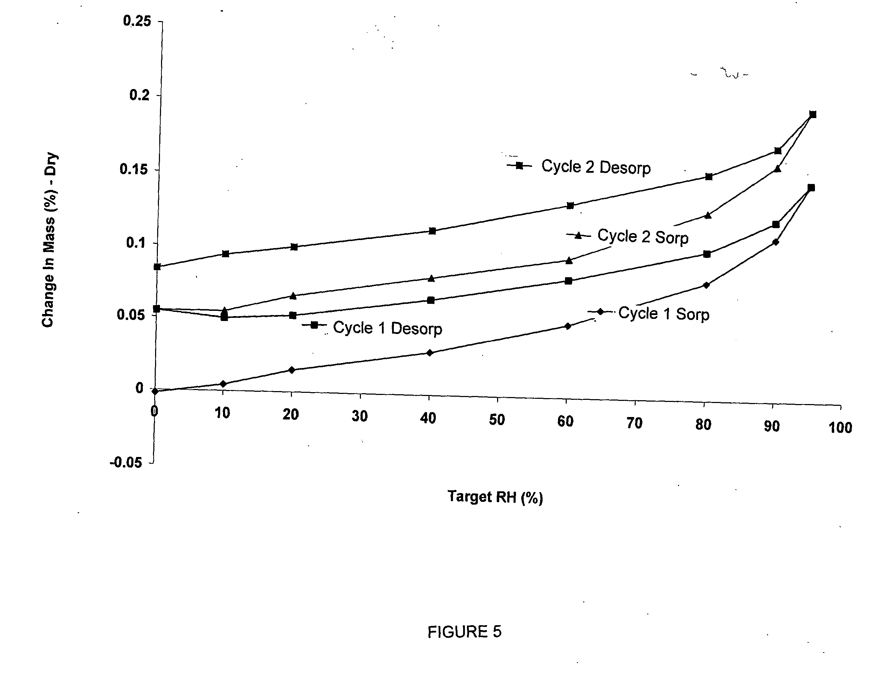

The DVS isotherms of the crystallized form B of dronedarone base are represented on Figure 5, wherein the change in dry mass (expressed in percentages) is plotted against the target relative humidity (RH, expressed in percentages). On Figure 5, « Sorp » and « Desorp » respectively designate the humidity sorption and desorption of each cycle. These results show that the hygroscopicity of said form B is low.

The above data show that the crystallized form B of dronedarone base displays properties of stability and low hygroscopicity which makes this form particularly suitable for use in pharmaceutics. Compared to the other known crystalline forms of dronedarone base, form B is the thermodynamically most stable one at and around room temperature : form A transforms to form C upon heating, while form C is unstable at room temperature. Form B cannot spontaneously transform into another solid form during storage, which makes it particularly useful in pharmaceutical formulation.

### 2.5 : Stability studies

The stabilities of two crystalline forms of dronedarone base, namely form B according to the instant invention and form A described above, are studied in a climatic chamber (reference : Firlabo SP_BVEHF) at 50°C, with or without 80% relative humidity (RH). The duration time of the stability study is set up at 1 month. Samples are monitored in terms of appearance by microscope observation, chemical degradation by HPLC, crystalline form and phase change by XRPD (X-Ray Powder Diffraction, reference : Bruker D5000) and thermal behavior by DSC and TGA (references : TA Instrument Q100/Q1000 for DSC analyses and Q50/Q500 for TGA analyses).

HPLC is performed with a Waters 2487 apparatus, using as mobile phase a gradient of acetonitrile and pH 4.0 phosphate buffer (30/70 to 70/30, V/V, gradient).

The results in terms of chemical degradation are presented in table 8, which summarizes the content in degradation product measured in the samples. The results in table 8 represent percentages in impurities (impurities appearing after the storage for 15 to 30 days, i.e. total impurities after storage minus impurities already present at t = 0). The percentages correspond to the area under the peaks detected in chromatography.

**Table 8**

| | t=0 | t = 15 days | t = 15 days | t = 30 days | t = 30 days |
|---|---|---|---|---|---|
| | | 50°C | 50°C/80%RH | 50°C | 50°C/80%RH |
| Form A | 0.00 | 0.12 | 0.09 | 0.15 | 0.13 |
| Form B | 0.00 | 0.05 | 0.05 | 0.06 | 0.06 |

These results demonstrate that the crystallized form B of dronedarone base is more stable than form A under the same storage conditions.

A yellow coloration is visually detected for the form A after 15 and 30 days at 50°C/80%RH, as well as an agglomeration of the powder, while no change is detected for the form B.

XRPD analyses show the appearance of some peaks of form B in the form A sample after 15 days at 50°C/80%RH, while no change is detected for the form B in all tested conditions and up to 30 days.

Thermal analyses do not reveal changes by TGA for both forms, while an additional endothermic peak in the DSC spectrum is detected for the sample of form A stored at 50°C/80%RH. This may be due to the presence of form B in the form A sample after storage, already observed by XRPD. No change in the DSC spectra is observed for the samples of form B, whatever their storage conditions.

These results show that form B is more stable than form A in terms of crystalline phase.

## Claims

1. Crystallized form B of dronedarone base, **characterized in that** its powder X-ray diffractogram shows the following characteristic peaks expressed as interplanar distances at approximately: 18.6 ; 4.75 ; and 4.23 Å.

2. The crystallized form of dronedarone base according to claim 1, **characterized in that** it shows the following characteristic peaks expressed as interplanar distances at approximately : 18.6 ; 7.97 ; 6.45 ; 5.73 ; 4.75 ; 4.71 ; 4.65 ; 4.23 ; 4.18 ; and 3.90 Å.

3. The crystallized form of dronedarone base according to claim 1, **characterized by** the powder X-ray diffractogram according to Figure 1.

4. Crystallized form B of dronedarone base, having a melting point of 72°C ± 2°C, corresponding to a DSC peak temperature.

5. Crystallized form B of dronedarone base, whose characteristics peaks in Raman spectroscopy are found at the following wavelengths at approximately : 2926; 2873 ; 1624 ; 1592 ; 1565 ; 1375 ; 1314 ; and 1156 cm⁻¹.

6. Process for the preparation of the crystallized form B of dronedarone base according to any of claims 1 to 5, comprising the following steps starting from dronedarone base:
1) preparing the crystallized form A of dronedarone base, and
2) converting said form A into form B by one the following steps :
a) maturation of a suspension of form A,
b) addition of an antisolvent to a solution of form A,
c) evaporation at elevated temperature of a solution or suspension of form A, or
d) cooling of a solution of form A.

7. The process according to claim 6, wherein step a) comprises the maturation of a suspension of form A in a solvent or mixture of solvents chosen from water, water/methanol, ethanol, 1-propanol, 2-propanol, 2-butanol, i-butanol, t-butanol, 1-pentanol, acetone, methyl ethyl ketone, i-butyl methyl ketone, ethyl formate, methyl acetate, propyl acetate, i-propyl acetate, butyl acetate, i-butyl acetate, tetrahydrofuran, diisopropyl ether, methyl t-butyl ether, toluene, acetonitrile, methylene chloride, 1,4-dioxane and chloroform.

8. The process according to claim 6, wherein step b) is carried out by dissolving form A in a solvent, then adding an antisolvent.

9. The process according to claim 8, wherein said solvent is chosen from diisopropyl ether and methyl tert-butyl ether.

10. The process according to claim 8 or claim 9, wherein said antisolvent is chosen from alkane solvents, such as n-heptane.

11. The process according to claim 6, wherein step c) is carried out by dissolving or suspending form A in a solvent at elevated temperature, then stirring the solution and allowing the solvent to evaporate from the stirred solution at this elevated temperature.

12. The process according to claim 11, wherein said solvent is chosen from water or a mixture of water and methanol.

13. The process according to claim 11 or claim 12, wherein the temperature is about 40°C or more, such as between about 40°C and about 65°C.

14. The process according to claim 6, wherein step d) is carried out by dissolving form A in a solvent at elevated temperature, then slowly cooling the solvent to a low temperature.

15. The process according to claim 14, wherein said solvent is chosen from the solvents and mixtures of solvents defined in claim 7, optionally in the presence of an antisolvent such as n-heptane.

16. The process according to claim 14 or claim 15, wherein said solvent is chosen from tetrahydrofuran, diisopropyl ether or a mixture diisopropyl ether/n-heptane.

17. The process according to any of claims 14 to 16, wherein form A is dissolved in a solvent at a tremperature comprised between about 40°C and about 80°C, and then the solvent is cooled to a temperature of about 10°C in about 24 hours.

18. Process for the preparation of the crystallized form B of dronedarone base according to any of claims 1 to 5, comprising a step of adding seeds of form B to a reaction medium comprising a solution or suspension of dronedarone base.

19. A process for the purification of dronedarone base, which comprises a step for crystallizing phase B of dronedarone base.

20. Pharmaceutical composition comprising the crystalline form B of dronedarone base according to any of claims 1 to 5 as active ingredient, and at least one pharmaceutically acceptable excipient.
